# EUROPEAN PATENT APPLICATION

(11) **EP 4 162 860 A1**
(43) Date of publication of application: **12.04.2023**
(21) Application number: 21201608.3
(22) Date of filing: 08.10.2021
(51) Int. Cl.: A61B 1/005, A61B 1/00

(54) **AN ENDOSCOPE**

(71) Applicant: Ambu A/S, 2750 Ballerup (DK)
(72) Inventor: PEDERSEN, Søren, 2880 Bagsværd (DK); SILBERMANN, Victor, 8653 Them (DK)
(74) Representative: AWA Denmark A/S

(57) **Abstract**

An endoscope comprising a proximal handle and an insertion cord extending from said towards a distal end of said endoscope. The insertion cord comprises a main tube having a proximal main tube end and a distal main tube end, and a bending section (7) connected to said distal main tube end. The bending section comprises (7) a through pull wire passage terminated at the proximal end in a receptacle provided in an inner wall of the bending section (7) and adapted to receive a distal end of a guide tube (193,194) for the pull wire. The bending section (7) comprises a glue passage (710) adapted to provide fluid flow for an adhesive to said receptacle during assembly of the endoscope. The glue passage (710) is at least partially filled with said adhesive in a cured state in the assembled endoscope.

## Description

The present disclosure relates to endoscopes, more specifically to an endoscope comprising an articulated bending section.

Endoscopes are well known devices for visually inspecting inaccessible places such as human body cavities. Typically, the endoscope comprises an elongated insertion cord with a handle at the proximal end as seen from the operator and visual inspections means, such as a built-in camera, at the distal end of the elongated insertion cord. This convention of distal and proximal, proximal being the end closest to the operator and distal being the end remote from the operator, as used above for the endoscope in general will, where applicable, be adhered to for all parts throughout this description. Electrical wiring for the camera and other electronics such as LED lighting run along the inside of the elongated insertion tube from the handle to the tip at the distal end. Instead of using cameras, endoscopes may also be fibre-optic, in which case the optical fibres run along in-side of the elongated insertion tube. Also, a working channel may run along the inside of the insertion cord from the handle to the tip, e.g. allowing liquid to be removed from the body cavity or allowing the insertion of surgical instruments or the like into the body cavity.

Furthermore, in order to be able to manoeuvre the endoscope inside the body cavity, the distal end of the endoscope may comprise bending section, i.e. a section with increased flexibility, such an articulated tip part allowing the operator to bend this section. Typically, this is done by tensioning or slacking pull wires in a guide tube also running along the inside of the elongated insertion cord from the articulated tip part to a control mechanism with a control knob in the handle in an arrangement commonly known as a Bowden cable.

The pull wire running along the inside of the guide tube of a Bowden cable normally extends with a predetermined length over either end, allowing an operating member to be attached to a free end, in the following referred to as the proximal end, and an operated member to be attached to the other free end, in the following referred to as the distal end. When the ends of the guide tube are held stationary, movement of the proximal end of the pull wire with respect to the guide tube is transmitted to the distal end as a corresponding movement of the distal end of the pull wire with respect to the guide tube, so as to effect a movement of the operated member.

When assembling the endoscope, care needs to be taken that the guide tubes are placed and maintained in good contact with the bending section as otherwise the pull wires will not function properly. During assembly the pull wires are first attached to the bending section and then threaded though the guide tubes, that is to say the guide tubes are pulled over the free ends of the pull wires until they engage the bending section. In a subsequent step, the guide tubes and pull wires, along with e.g. a working channel tube, optical illumination fibres, and electrical wiring are placed in a main tube, which is pulled over this preassembled arrangement until the end of the main tube engages and can be secured to the bending section. In this respect, US2020/0196835, incorporated herein by reference, discloses a bending section where the proximal end segment is adapted to receive the distal end of a main tube. The main tube is secured with respect to the bending section by means of an adhesive introduced between the main tube and the bending section and in holes lateral openings. US2020/0196835 does not disclose anything about pull wires and guide tubes. Another bending section is disclosed in US2015/0335227, also incorporated herein by reference. US2015/0335227 has passages for pull wires but does not disclose any details on the pull wires or possible guide tubes.

Based on this prior art it is the object of the disclosure to provide an endoscope with a bending section which, in particular during assembly, ensures that the guide tubes remain in their intended place and in good contact with the bending section, thus facilitating assembly. Facilitating the assembly in itself is important, because current trends go towards disposable i.e. single use endoscopes which by nature need to be low cost in materials and other manufacturing costs such as wages.

According to a first aspect of the disclosure, this object is achieve by an endoscope comprising a proximal handle and an insertion cord extending from said towards a distal end of said endoscope, said insertion cord comprising a main tube having a proximal main tube end and a distal main tube end, and a bending section connected to said distal main tube end, wherein said bending section comprises a through pull wire passage terminated at the proximal end in a receptacle provided in an inner wall of the bending section and adapted to receive a distal end of a guide tube for the pull wire, and wherein said bending section comprises a glue passage adapted to provide fluid flow for an adhesive to said receptacle during assembly of the endoscope, and wherein said glue passage is at least partially filled with said adhesive in a cured state in the assembled endoscope. This use of a receptacle allows the distal end of the guide tube to be held in a well-defined position, where it may be at least temporarily secured by an adhesive, so as not to be dislocated if hit by the main tube during assembly forces. The glue passage not only facilitate the introduction of the adhesive but when adhesive remains and cures in the passage the adhesive forms a hard, radial protrusion mechanically locking the guide tube to the bending section.

According to a second aspect of the disclosure the object is achieved by a method in assembling an endoscope, said method comprising providing a bending section comprising a through pull wire passage terminated in a receptacle provided in an inner wall of the bending section and adapted to receive a distal end of a guide tube for the pull wire, and a glue passage adapted to provide fluid flow for an adhesive to said receptacle during assembly of the endoscope, inserting the distal end of the guide tube into the receptacle, and introducing an amount of adhesive into said glue passage sufficient to at least partially filled with said adhesive in a cured state in the assembled endoscope, curing the adhesive. This use of a receptacle allows the distal end of the guide tube to be readily held in a well-defined position during assembly, where it may be at least temporarily secured by an adhesive, so as not to be dislocated if hit by the main tube during assembly forces. Less caution is thus needed during assembly. This advantage is further improved because the glue passage not only facilitate the introduction of the adhesive but when adhesive remains and cures in the passage the adhesive forms a hard, radial protrusion mechanically locking the guide tube to the bending section.

According to a third aspect of the disclosure the object is achieved by a system comprising a display unit and an endoscope according to the first aspect of the disclosure, connectable to said display unit. Making the disposable endoscope in a more cost-efficient manner allows the overall price of the system to be kept down.

According to an embodiment of the first aspect of the disclosure, the bending section comprises a plurality of articulated segments interconnected by hinges, said plurality of articulated segments comprising a proximal end segment, at least one intermediate segment, and a distal end segment, where the proximal end segment, the at least one intermediate segment and the distal end segment each comprise at least one pull wire passage forming part of said through pull wire passage, and where the receptacle is provided within the proximal end segment. Such an articulated bending section may readily be moulded as one single integral piece, e.g. by injection moulding.

According to an embodiment of the first aspect of the disclosure, each of said a proximal end segment, at least one intermediate segment, and a distal end segment comprise a pair of pull wire passages each passage of each pair being arranged diametrically opposite each other within the respective segment. Having two diametrically opposite pull wires is an efficient way of providing a bending section that may bend in more than one direction.

According to an embodiment of the first aspect of the disclosure, a wire is secured to the distal end segment beyond said pair of passages, so as to provide two free ends, and each of said free ends is accommodated in one of the passages of said pair of passages in the proximal end segment, the at least one intermediate segment, and the distal end segment. This is advantageous as it facilitates the assembly process where only a single wire needs to be handled, i.e. threaded through the bending section (and back). This single wire once secured presents two free ends *de facto* constituting two individual pull wires.

According to an embodiment of the first aspect of the disclosure, the proximal end segment comprises a main tube reception sector, a transition sector and a pull wire passage sector through all of which a central lumen extends, where the cross-sectional area of the central lumen deceases from the proximal end, via the transition sector, towards the pull wire sector, so as to provide an abutment surface for the main tube, and where said glue passage is provided in said transition sector away from said abutment surface. This allows the adhesive to be injected at a location where it will not interfere with the subsequent insertion of the main tube into the proximal end section during assembly. This is in particular the case when, according to an embodiment of the first aspect of the disclosure, the transition sector comprises a cut-out provided the abutment surface.

The disclosure will now be made in greater detail based on nonlimiting exemplary embodiments and with reference to the drawings, on which:
Fig. 1 shows a system comprising a display unit and an endoscope according to the disclosure,
Fig. 2 shows an exploded view of the endoscope according to the disclosure,
Fig. 3 shows a top view of a bending section according to the disclosure,
Fig. 4 shows a side view of a bending section of Fig. 3,
Fig. 5 shows an isometric view from the proximal end of the bending section of Fig. 3,
Fig. 6 shows an isometric view corresponding to Fig. 5 but with guide tubes in place,
Fig. 7 shows an isometric view corresponding to Fig. 6 but with the main tube in place,
Fig. 8 shows the bending section of Fig. 3 from the proximal end, and
Fig. 9 is a cross-sectional view around the interface between the main tube and the bending section.

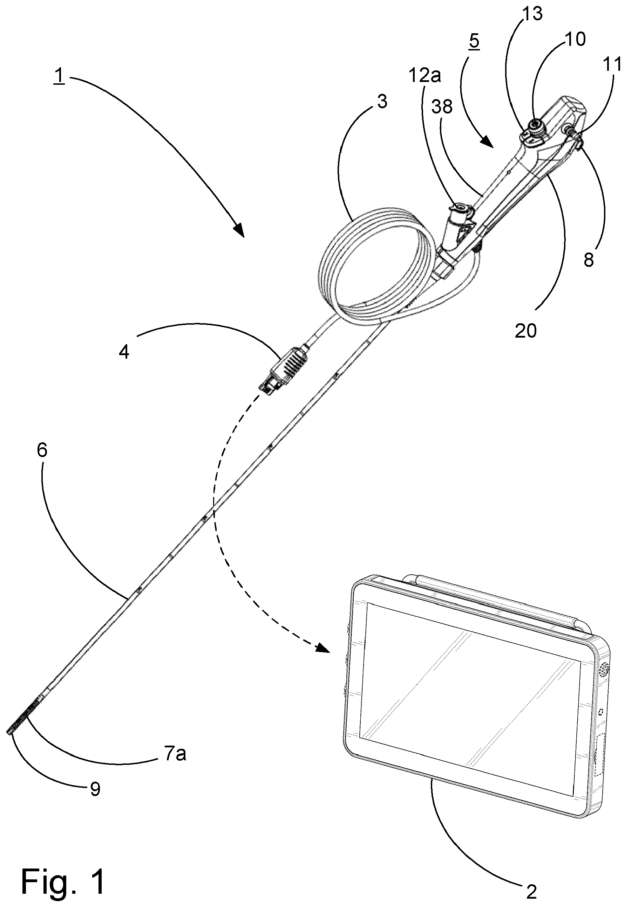

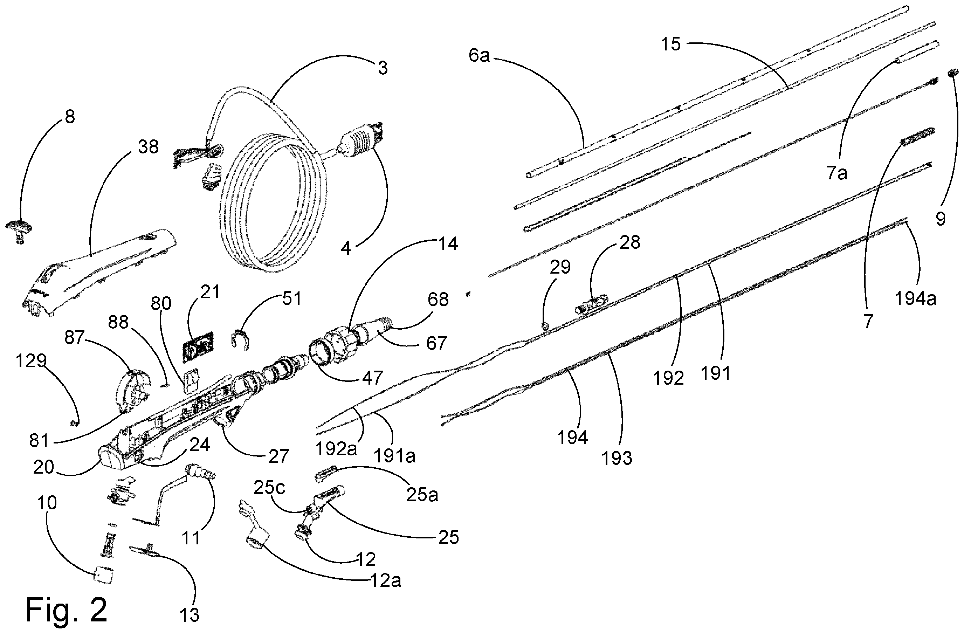

Turning first to Fig. 1, a system comprising an endoscope 1 and a display unit 2 is shown. The endoscope 1 may be connected to the display unit 2 by means of a cable 3 with a suitable connector 4. The endoscope 1 is preferably disposable i.e. intended to be thrown away after use on one single patient, whereas the display unit 2 may be used multiple times with different reusable endoscopes. The endoscope 1 is an insertion endoscope comprising a handle 5 at the proximal end, and an insertion cord 6 extending from the handle 5 towards the distal end of the endoscope 1. The handle preferably comprises two major constituent part, i.e. main handle housing part 20 and a lid part 38. At the distal end of the insertion cord 6 a bending section which may be controlled by an operating member 8 is provided. As can be seen from Figs. 2 and 2b the bending section comprises a bending section body 7 beneath a flexible cover 7a. In turn at the distal end of the bending section 7 a tip housing 9 with the image capture device, lenses, illumination, the distal port of a working and/or suction cannel etc. of the endoscope 1 is provided. At the distal end the working channel will normally double as both working channels for tools and suction channel. The handle 5 normally also comprises a suction activation button 10 for activating the suction though the suction channel to an external vacuum source (not shown) via a suction connector 11. The handle may also have a tool insertion port 12 (cf. Fig 2) with a cap 12a for the insertion of an external tool through the handle and the working channel so as to emerge from the distal working channel port at the tip housing 9. Also, the handle 5 may be provided with buttons for activation electrical switches 13 controlling function of the image capture, such as taking of still images.

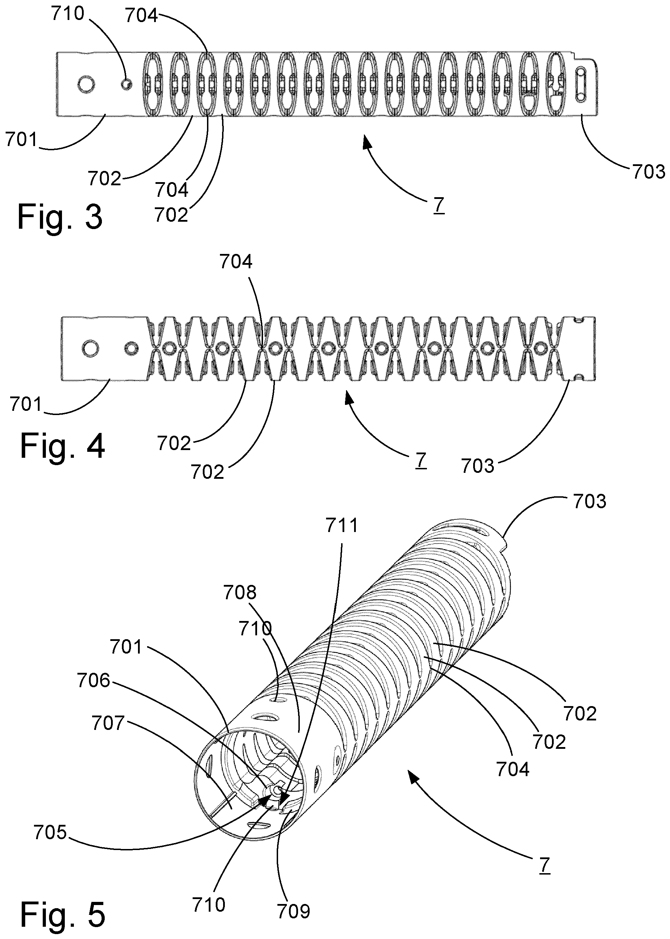

The bending section 7 is show in greater detail in Figs. 3 to 8. The bending section comprises a plurality of articulated segments 701, 702, 703 interconnected by thin hinges 704, arranged diametrically opposite each other. The plurality of articulated segments 701, 702, 703 comprise a proximal end segment 701, at least one intermediate segment 702, and a distal end segment 703. The proximal end segment 701, the at least one intermediate segment 702 and the distal end segment 703 each comprise at least one pull wire passage 705 forming part of complete pull wire passage for a pull wire 191, 192 from the proximal end segment through to the distal end segment 703.

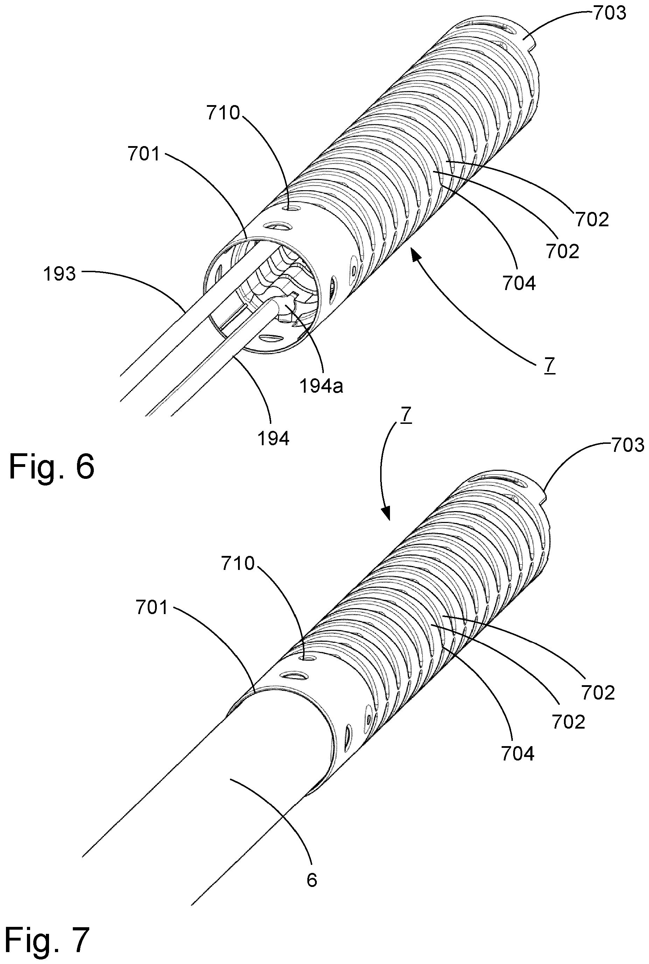

At the proximal end the pull wire passage extends into a wider receptacle 706 in the proximal end segment 701. The receptacle 706 is preferably cylindrical or slightly conical with a diameter adapted to receive and hold a distal end of a guide tube 193,194, as can be seen in Fig. 6. As can be derived from Figs. 5 or 6, the proximal end segment 701 comprises a central lumen. The central lumen comprises main tube reception sector 707, a transition sector 712 and a pull wire passage sector 708 through all of which the central lumen extends. The cross-sectional area of the central lumen decreases from the main tube reception sector 707, via the transition sector 712, to the pull-wire passage sector 708, so that the lumen of the main tube reception sector 707 is large enough to receive the distal end of the main tube 6 whereas the central lumen of the pull wire sector 708 will essentially only be able to accommodate the working channel tube 15 and electrical wires etc. to the tip part 9. The transition sector 712 extends in between from an abutment surface 709 for the distal end of the main tube 6 to the bottom of the guide wire receptacle 706.

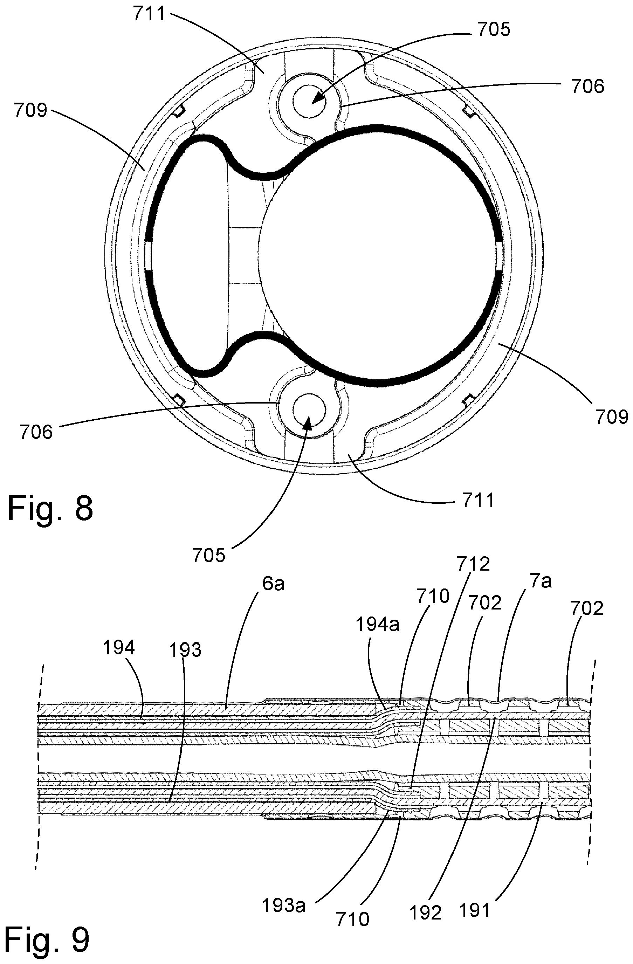

For optimum bending force the pull wire passages 705 are preferably provided diametrically opposite each other as close to the outer surface of the segments 701, 702, 703 as possible. During manufacture and subsequently the guide tubes will normally be elastically deformed slightly in an S-curve 194a when surrounded by the main tube 6, as best seen in Fig. 9. This is because the thickness of the wall of the main tube 6 provides a smaller lumen than the central lumen of the main tube insertion sector 707 and the transition sector. This deformation would potentially dislocate the distal ends of the guide tubes 193, 194 if no countermeasures are taken. This is at least partially avoided because the distal ends of the guide tubes are accommodated in the receptacles 706. This securing can however be further improved. So when, during assembly, the guide tubes 193, 194 have been pulled over the pull wires 191,192 and their distal ends inserted into the receptacles 706, they are secured using an adhesive, e.g. a UV setting polymer cured by irradiation with ultraviolet light, but other adhesives may of course also be used.

The adhesive is introduced in glue passages 710 and in any gap between the distal ends of the guide tubes 193, 194 and the walls of the receptacles 706. Preferably, glue passages are adapted to allow the adhesive to be introduced into the glue passages 710 and flow into the gaps via the glue passages 710. The amount of adhesive introduced is selected so that adhesive will remain in the glue passages 710. The adhesive is then allowed to set or harden, e.g. by UV curing. The adhesive in the glue passages will when cured provide rigid and hard radial protrusions mechanically locking the distal ends of the guide tubes 193, 194 with respect to the bending section 7 by means of the glue passages 710. Thus, when in a subsequent step, the main tube 6 is pulled or pushed over the guide tubes 193, 194 and into the main tube receiving sector 707 of the bending section, the occurring forces bending the guide tubes into elastically the S-shape or similar curve will not dislocate the distal ends of the guide tubes 193, 194. Subsequently, the main tube is then also secured in the main tube receiving sector 707 of the bending section 7 by means of an adhesive.

To accommodate for the S-curve 194a of each of the guide tubes 193, 194 a cut-out 711 is provided in the abutment surface 709 in conjunction with each guide tube receptacle 706. The glue passages 710 are at least partially located within said cut-outs 711 so as to allow adhesive to flow into this gap and not only into the receptacle 706. The cut-outs 711 have a size adapted to ensure that adhesive does not flow beyond the abutment surface 709 and into the main tube receiving sector 707 where it could obstruct the subsequent insertion of the distal end of the main tube 6.

## Claims

1. An endoscope comprising a proximal handle and an insertion cord extending from said handle towards a distal end of said endoscope, said insertion cord comprising a main tube having a proximal main tube end and a distal main tube end, and a bending section connected to said distal main tube end,
wherein said bending section comprises a through pull wire passage terminated at the proximal end in a receptacle provided in an inner wall of the bending section and adapted to receive a distal end of a guide tube for the pull wire,
and wherein said bending section comprises a glue passage adapted to provide fluid flow for an adhesive to said receptacle during assembly of the endoscope, and
wherein said glue passage is at least partially filled with said adhesive in a cured state in the assembled endoscope.

2. An endoscope according to claim 1, wherein the bending section comprises a plurality of articulated segments interconnected by hinges, said plurality of articulated segments comprising a proximal end segment, at least one intermediate segment, and a distal end segment,
where the proximal end segment, the at least one intermediate segment and the distal end segment each comprise at least one pull wire passage forming part of said through pull wire passage,
and where the receptacle is provided within the proximal end segment.

3. An endoscope according to any one of the preceding claims, wherein each of said a proximal end segment, at least one intermediate segment, and a distal end segment comprise a pair of pull wire passages each passage of each pair being arranged diametrically opposite each other within the respective segment.

4. An endoscope according to claim 3, wherein a wire is secured to the distal end segment beyond said pair of passages, so as to provide two free ends, and each of said free ends is accommodated in one of the passages of said pair of passages in the proximal end segment, the at least one intermediate segment, and the distal end segment.

5. An endoscope according to any one of claims 2 to 4, wherein the proximal end segment comprises a main tube reception sector, a transition sector and a pull wire passage sector through all of which a central lumen extends,
wherein the cross-sectional area of the central lumen deceases from the proximal end, via the transition sector, towards the pull wire sector, so as to provide an abutment surface for the main tube, and wherein said glue passage is provided in said transition sector away from said abutment surface.

6. An endoscope according to claim 5 wherein the transition sector comprises a cut-out provided the abutment surface.

7. A method in assembling an endoscope, said method comprising
providing a bending section comprising a through pull wire passage terminated in a receptacle provided in an inner wall of the bending section and adapted to receive a distal end of a guide tube for the pull wire, and a glue passage adapted to provide fluid flow for an adhesive to said receptacle during assembly of the endoscope,
inserting the distal end of the guide tube into the receptacle, and introducing an amount of adhesive into said glue passage sufficient to at least partially filled with said adhesive in a cured state in the assembled endoscope,
curing the adhesive.

8. System comprising a display unit and an endoscope according to any one of claims 1-6 connectable to said display unit.
